# EUROPEAN PATENT SPECIFICATION

(11) **EP 3 061 400 B1**
(45) Date of publication and mention of the grant of the patent: **26.12.2018**
(21) Application number: 15833184.3
(22) Date of filing: 15.04.2015
(51) Int. Cl.: A61B 8/08, A61B 8/12, A61B 1/00, A61B 1/005, A61B 1/015, A61B 1/018

(54) **ULTRASOUND ENDOSCOPE SYSTEM**
ULTRASCHALLENDOSKOPSYSTEM
SYSTÈME D'ENDOSCOPE À ULTRASONS

(30) Priority: 22.08.2014 JP 2014169442
(43) Date of publication of application: 31.08.2016
(73) Proprietor: Olympus Corporation, Tokyo 192-8507 (JP)
(72) Inventor: TSURUTA, Teppei, Hachioji-shi, Tokyo 192-8507 (JP)
(74) Representative: Gunzelmann, Rainer
(86) International application number: PCT/JP2015/061632
(87) International publication number: WO 2016/027508

(56) References cited:
- JP-A- 2001 224 594
- JP-A- 2001 224 594
- JP-A- 2009 254 468
- US-A1- 2011 238 006
- US-A1- 2011 282 209

## Description

### Technical Field

The present invention relates to an ultrasound endoscope system.

### Background Art

In recent years, elastography for displaying hardness of living tissue has been put to practical use in ultrasound observation using an ultrasound diagnostic apparatus (see, for example, Japanese Patent Application Laid-Open Publication No. 2012-81295 (Patent Literature 1)). In the elastography technique, it is possible to measure change (displacement) of a deformed state of living tissue, for example, by causing a pressing state of an ultrasound probe against an organ, which can be detected by ultrasound, to switch among two or more states, and construct an elastography image from strain obtained by spatially differentiating the displacement.

Application of such an elastography technique to various ultrasound observation apparatuses is expected. For example, if the elastography technique is applied to an ultrasound endoscope having an ultrasound probe at a distal end of an insertion portion, it is possible to cause a rate of detecting a lesion in a deep organ to be improved.

In order to detect a lesion by an elastography function, however, it is necessary to perform periodical pressing of the ultrasound probe against tissue in a body cavity, and the like.

Here, as a method for periodically pressing the ultrasound probe in the body cavity, for example, a method of utilizing beats of a heart is conceivable. However, since change in the pressing state in observation utilizing beats is slight, there is a possibility that it is difficult to acquire a stable and favorable elastography image.

Further, as the method for periodically pressing the ultrasound probe in the body cavity, for example, a method of a surgeon or the like manually causing the insertion portion to move back and forth is conceivable. However, the insertion portion of an endoscope is generally long, and, furthermore, it has flexibility. Therefore, even if the insertion portion is caused to move back and forth, much of an amount of the movement is absorbed by the flexibility and the like midway. Thus, in this case also, there is a possibility that it is difficult to acquire a stable and favorable elastography image.

To cope with this, it is conceivable to provide a pressing mechanism for mechanically pressing the body cavity at a distal end portion of the endoscope. However, there is a possibility that providing such a mechanism at the distal end portion brings about upsizing, structure complication and the like of the distal end portion.

JP 2001 224594 A concerns an ultrasonic endoscope that emits an ultrasonic wave by an ultrasonic transducer formed at the tip of the ultrasonic endoscope, receives the reflection signal from a living body, and applies a displacement to the biological tissue. An ultrasonic observation device gains a hardness image by an operation using the reflection signal from the biological tissue which is displaced, and displays it on a monitor. The hardness of the biological tissue can be measured by use of the ultrasonic endoscope and used for diagnosis.

US 2011/282209 A1 concerns an ultrasound observation apparatus including an ultrasound probe portion capable of two-dimensional scanning of an ultrasound beam; a transmission/reception control section for controlling a transmission/reception direction of the ultrasound beam by the ultrasound probe portion; a B-mode image calculation section for generating a B-mode image on a scanning plane based on a result of scanning of the ultrasound beam; a storage section for storing a predetermined sample image determined according to a shape of a treatment instrument in a case where a central axis of the treatment instrument for performing a treatment on a subject and the scanning plane agree with each other; and a correlation calculation section for calculating a correlation value between the B-mode image and the sample image, wherein the transmission/reception control section moves the scanning plane such that the correlation value becomes the maximum.

US 2011/238006 A1 concerns a needleless injection system including a tissue tensioner and a fitting for attaching to a distal end of a shaft. Embodiments include an elastic sleeve sized to fit under tension about the distal end of a flexible scope, such as an endoscope; a non-metal, polymeric tube-like device being optionally attached to the elastic sleeve for delivering a therapeutic fluid to a treatment site within a patient wherein the elastic sleeve may include an integral balloon (tissue tensioner) feature that can be used to position the injection orifice of the tube-like device proximate a treatment area.

The present invention has been made in view of the above situation, and an object is to provide an ultrasound endoscope system with an ultrasound endoscope making it possible to obtain a favorable elastography image by a simple configuration without causing a distal end portion to be upsized.

### Disclosure of Invention

### Means for Solving the Problem

An ultrasound endoscope system according to a first aspect of the present invention includes the features of claim 1. An ultrasound endoscope system according to a second aspect of the present invention includes the features of claim 6.

### Brief Description of the Drawings

Fig. 1 relates to a first embodiment of the present invention and is a configuration diagram of an ultrasound endoscope system;
Fig. 2 relates to the first embodiment of the present invention and is an explanatory diagram schematically showing a relationship between an ultrasound probe and a stomach wall when water is not fed;
Fig. 3 relates to the first embodiment of the present invention and is an explanatory diagram schematically showing a relationship between the ultrasound probe and the stomach wall when water is fed;
Fig. 4 relates to the first embodiment of the present invention and is a perspective view showing a distal end portion;
Fig. 5 relates to the first embodiment of the present invention and is an end face view showing the distal end portion;
Fig. 6 relates to a second embodiment of the present invention and is a schematic configuration diagram of a liquid feeding apparatus for ultrasound endoscope;
Fig. 7 relates to a first modification of the second embodiment of the present invention and is a schematic configuration diagram of a liquid feeding apparatus for ultrasound endoscope;
Fig. 8 relates to a second modification of the second embodiment of the present invention and is a schematic configuration diagram of a liquid feeding apparatus for ultrasound endoscope;
Fig. 9 relates to the second modification of the second embodiment of the present invention and is a time chart showing pump generated pressure;
Fig. 10 relates to a third modification of the second embodiment of the present invention and is a schematic configuration diagram of a liquid feeding apparatus for ultrasound endoscope;
Fig. 11 relates to a fourth modification of the second embodiment of the present invention and is a schematic configuration diagram of a liquid feeding apparatus for ultrasound endoscope;
Fig. 12 relates to a fifth modification of the second embodiment of the present invention and is a schematic configuration diagram of a liquid feeding apparatus for ultrasound endoscope;
Fig. 13 relates to a third embodiment of the present invention and is an exploded perspective view showing a distal end portion and an adapter;
Fig. 14 relates to the third embodiment of the present invention and is a cross-sectional view showing main portions of the distal end portion equipped with the adapter;
Fig. 15 relates to a first modification of the third embodiment of the present invention and is an exploded perspective view showing a distal end portion and an adapter;
Fig. 16 relates to the first modification of the third embodiment of the present invention and is a cross-sectional view showing main portions of the distal end portion equipped with the adapter;
Fig. 17 relates to a second modification of the third embodiment of the present invention and is an exploded perspective view showing a distal end portion and an adapter;
Fig. 18 relates to the second modification of the third embodiment of the present invention and is a cross-sectional view showing main portions of the distal end portion equipped with the adapter;
Fig. 19 relates to a fourth embodiment of the present invention and is a block diagram showing a configuration of an ultrasound endoscope system;
Fig. 20 relates to the fourth embodiment of the present invention and is a flowchart showing an elastographic image generation process routine; and
Fig. 21 relates to a first modification of the fourth embodiment of the present invention and is a block diagram showing a configuration of an ultrasound endoscope system.

### Best Mode for Carrying Out the Invention

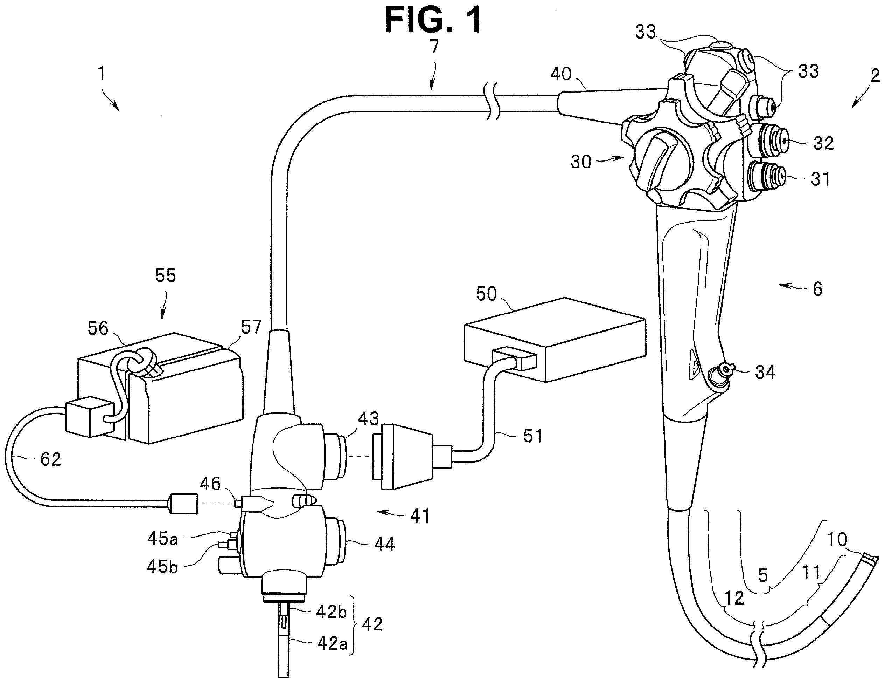

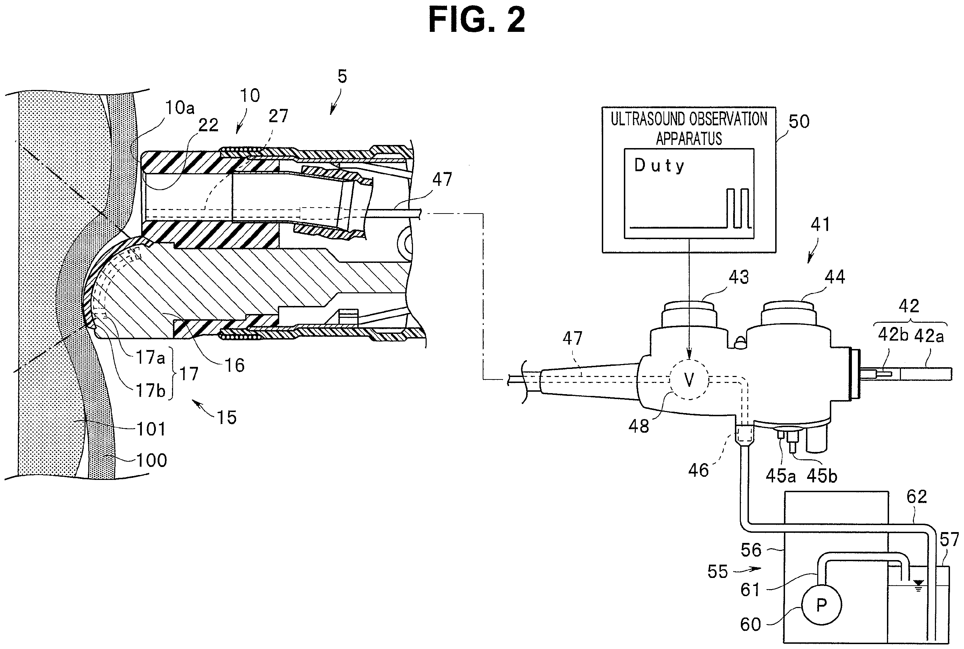

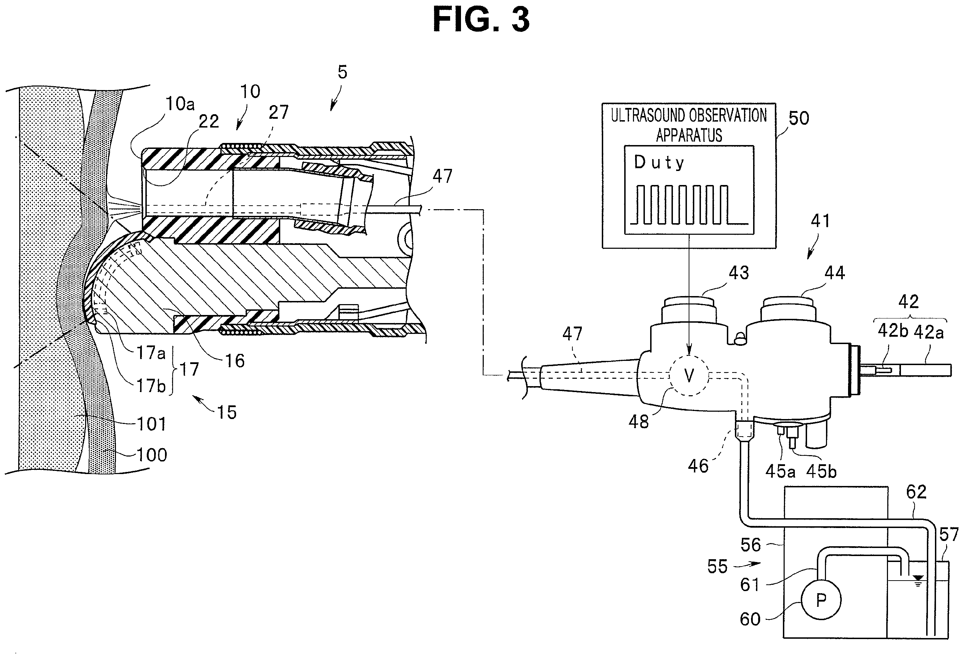

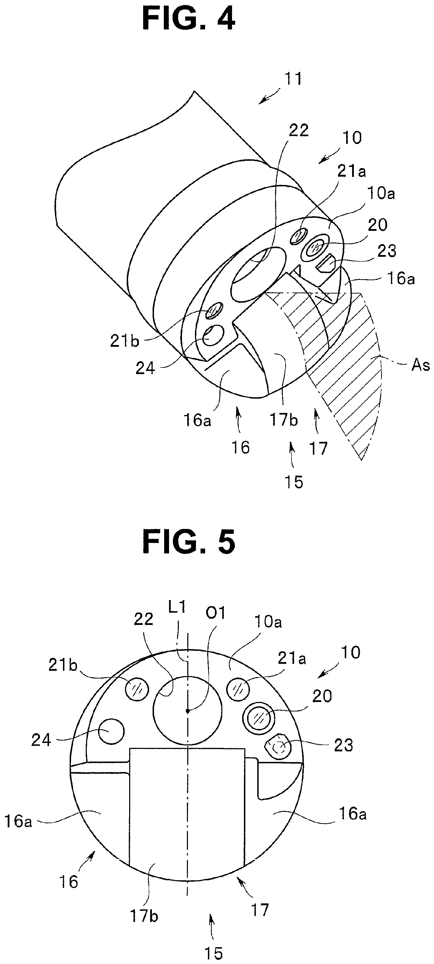

Embodiments of the present invention will be described below with reference to drawings. Figs. 1 to 5 relate to a first embodiment of the present invention. Fig. 1 is a configuration diagram of an ultrasound endoscope system; Fig. 2 is an explanatory diagram schematically showing a relationship between an ultrasound probe and a stomach wall when water is not fed; Fig. 3 is an explanatory diagram schematically showing a relationship between the ultrasound probe and the stomach wall when water is fed; Fig. 4 is a perspective view showing a distal end portion; and Fig. 5 is an end face view showing the distal end portion.

An ultrasound endoscope system 1 of the present embodiment shown in Fig. 1 has an ultrasound endoscope (hereinafter also referred to simply as an endoscope) 2, for example, for a stomach and a duodenum. The endoscope 2 is configured, being provided with an elongated insertion portion 5 to be inserted into a body cavity, an operation portion 6 provided at a proximal end of the insertion portion 5, and a universal cord 7 extending from the operation portion 6.

The insertion portion 5 is provided with a distal end rigid portion 10, a bending portion 11 located at a proximal end of the distal end rigid portion 10, and a long and flexible tube portion 12 with a small diameter which is located at a proximal end of the bending portion 11 and leads to the operation portion 6, and these are connectedly arranged in that order from a distal end side to constitute main portions.

As shown in Figs. 2 to 5, the distal end rigid portion 10 is provided with an ultrasound unit 15 for obtaining acoustic image information by ultrasound. The ultrasound unit 15 of the present embodiment is a convex-shaped ultrasound unit, and the ultrasound unit 15 is configured having a nose piece 16, which is a case, and an ultrasound probe 17 as an ultrasound observation portion.

The nose piece 16 has, for example, a tissue abutting face 16a forming a convex-shaped partial arc, and the tissue abutting face 16a projects further forward than a distal end face 10a of the distal end rigid portion 10 (see Figs. 4 and 5).

The ultrasound probe 17 is configured having a plurality of ultrasound transducers 17a arrayed in a convex-shaped partial arc and an acoustic lens 17b covering a front of the ultrasound transducers 17a (see Figs. 2 and 3). The ultrasound probe 17 is arranged at a substantial center of the tissue abutting face 16a of the nose piece 16 (see Figs. 4 and 5). Further, the acoustic lens 17b of the ultrasound probe 17 projects further forward than the distal end face 10a of the distal end rigid portion 10 together with the tissue abutting face 16a of the nose piece 16. Thereby, the ultrasound probe 17 can scan mainly living tissue and the like existing forward of in an endoscope insertion direction.

Further, for example, as shown in Figs. 4 and 5, an observation window 20 constituting an observation optical system, a pair of illuminating windows 21a and 21b constituting an illumination optical system, a suction/forceps opening 22 from which a treatment instrument such as a puncture needle is guided out, an air/water feeding nozzle 23 from which fluid such as air and water is ejected toward the observation window 20, and an auxiliary water feeding channel opening 24 as an opening portion from which fluid such as water is ejected forward of the distal end rigid portion 10 (that is, in a projecting direction of the ultrasound probe 17) are provided on the distal end face 10a of the distal end rigid portion 10.

Here, in order to arrange the treatment instrument guided out from the suction/forceps opening 22 in a scanning area As of the ultrasound probe 17, the suction/forceps opening 22 is arranged so that its central axis 01 is located on an extension line of a scanning direction (a center line L1) of the ultrasound probe 17 on the distal end face 10a of the distal end rigid portion 10 (see Fig. 5). Further, on the distal end face 10a of the distal end rigid portion 10, the observation window 20, the illuminating window 21a and the air/water feeding nozzle 23 are collectively arranged on one side of the suction/forceps opening 22, and the illuminating window 21b and the auxiliary water feeding channel opening 24 are collectively arranged on the other side of the suction/forceps opening 22. Thereby, the auxiliary water feeding channel opening 24 is arranged at a position offset relative to the scanning area As of the ultrasound probe 17 (see Fig. 4).

As shown in Fig. 1, an angle knob 30 for performing a bending operation of the bending portion 11 in a desired direction, an air/water feeding button 31 for performing an air feeding and water feeding operations, a suction button 32 for performing a suction operation, a plurality of buttons/switches 33 to which arbitrary functions among various functions related to the endoscope 2 can be allocated, and a treatment instrument insertion port 34 to be an entrance of a treatment instrument to be guided into a body are arranged on the operation portion 6. Here, in the present
embodiment, any of the buttons/switches 33 can be set, for example, as a switch for giving an instruction to start and end elastography observation using the ultrasound unit 15. Further, the treatment instrument insertion port 34 communicates with the suction/forceps opening 22 via a treatment instrument insertion channel (not shown) provided inside the insertion portion 5.

One end side of the universal cord 7 is arranged being connected to a side portion of the operation portion 6 via a bend preventing portion 40. On the other hand, a scope connector portion 41 is provided on an extension end, which is the other end side of the universal cord 7. At an end portion of the scope connector portion 41, a light source side connector 42 is provided which is attachable to and detachable from a light source apparatus not shown. On the light source side connector 42, proximal end portions of a light guide 42a and an air feeding tube 42b which extend from an insertion portion 5 side are projectingly provided, and an electrical contact not shown is arranged. Further, on one side portion of the scope connector portion 41, an ultrasound connector 43 attachable to and detachable from an ultrasound observation apparatus 50, an electrical connector 44 attachable to and detachable from a video processor not shown are provided side by side. Furthermore, on the other side portion of the scope connector portion 41, proximal end portions of a pressurized tube 45a and a water feeding tube 45b are projectingly provided, and an auxiliary water feeding pipe sleeve 46 attachable to and detachable from a liquid feeding apparatus for ultrasound endoscope 55 is also provided.

Here, as shown in Figs. 2 and 3, the auxiliary water feeding pipe sleeve 46 communicates with the auxiliary water feeding channel opening 24 via an auxiliary water feeding channel 47 as a fluid conduit. Further, for example, in the scope connector portion 41, an adjustment valve 48 as a first flow rate adjusting portion is interposed in a middle of the auxiliary water feeding channel 47. The adjustment valve 48 is configured, for example, with a normally closed electromagnetic solenoid valve. The adjustment valve 48 can control a flow rate of fluid flowing through the auxiliary water feeding channel 47 so that the flow rate is in an arbitrary state, by a valve opening time period being controlled by an arbitrary duty ratio. More specifically, for example, by the valve opening time period being periodically duty-controlled by an arbitrary duty ratio, it is possible for the adjustment valve 48 to cause the flow rate of the fluid flowing through the auxiliary water feeding channel 47 to switch among two or more arbitrary states (for example, it is possible to cause the flow rate to periodically switch between arbitrary two states). Note that the adjustment valve 48 can be provided inside the operation portion 6 or the like instead of being provided inside the scope connector portion 41. The flow rate stated here includes a state that the flow rate is zero.

As shown in Figs. 1 to 3, the liquid feeding apparatus for ultrasound endoscope 55 of the present embodiment is, for example, a pneumatic liquid feeding apparatus. The liquid feeding apparatus for ultrasound endoscope 55 is configured having a pump unit 56 which generates air pressure and a liquid feeding tank 57 which stores liquid (fluid) such as sterile water and degassed water. The pump unit 56 has a peristaltic pump 60, and the peristaltic pump 60 communicates with an upper part of the liquid feeding tank 57 via a pressurized tube 61. Further, one end of an auxiliary water feeding tube 62 is caused to face a bottom portion of the liquid feeding tank 57, and the other end of the auxiliary water feeding tube 62 is connected to the auxiliary water feeding channel 47 via the auxiliary water feeding pipe sleeve 46. In the liquid feeding apparatus for ultrasound endoscope 55, when the peristaltic pump 60 of the pump unit 56 is driven, air pressure which occurs in the peristaltic pump 60 is supplied to an upper part in the liquid feeding tank 57 via the pressurized tube 61. Internal pressure of the liquid feeding tank 57 is increased by the supply of the air pressure, and liquid such as degassed water stored in the liquid feeding tank 57 is guided out into the auxiliary water feeding channel 47 via the auxiliary water feeding tube 62 by the increase in the internal pressure. That is, in the present embodiment, the peristaltic pump 60 realizes a function as a fluid guiding-out portion.

As shown in Fig. 1, the ultrasound observation apparatus 50 is connected to the ultrasound connector 43 via an ultrasound connection cable 51. The ultrasound observation apparatus 50 drive-controls the ultrasound probe 17, and generates various kinds of ultrasound images based on an ultrasound echo signal (an ultrasound signal) received at the ultrasound probe 17 by the drive control. For example, the ultrasound observation apparatus 50 is capable of performing generation of a B-mode image in which amplification of the received ultrasound echo signal is associated with luminance, and the like.

Further, for example, an instruction to the effect that elastography observation is to be started is made through an operation of the buttons/switches 33 by a user or the like, the ultrasound observation apparatus 50 causes the peristaltic pump 60 to be driven, and adjusts a flow rate of fluid (for example, liquid such as degassed water) which flows through the auxiliary water feeding channel 47 through the control of the adjustment valve 48. Thereby, the flow rate of the fluid ejected from the auxiliary water feeding channel opening 24 changes, and pressing force living tissue receives by the ejection of the fluid changes. Since the ejection of the fluid presses the living tissue in the projecting direction of the ultrasound probe 17 in a vicinity of the ultrasound probe 17, the pressing force of the ultrasound probe 17 pressing the living tissue indirectly changes by the ejection of the fluid without causing the ultrasound probe 17 to move. As a result, it becomes possible for the ultrasound probe 17 to obtain an ultrasound signal in a different pressing state. Then, the ultrasound observation apparatus 50 measures change (displacement) of a deformed state of the living tissue based on the ultrasound signal in the different pressing state and generates an elastography image based on a displacement measurement result.

To describe a case of performing elastography observation of a pancreas 101 via a stomach wall 100 in order to perform examination of a tumor/lymph node metastasis of the pancreas 101 and the like as a specific example, referring to Fig. 2 and 3, the distal end rigid portion 10 of the endoscope 2 inserted in a body cavity is arranged at a position of pressing the stomach wall 100 and the pancreas 101 with predetermined pressing force via the ultrasound probe 17 first prior to observation. Further, the peristaltic pump 60 is driven by the ultrasound observation apparatus 50, and the adjustment valve 48 is duty-controlled. Thereby, for example, a state in which degassed water or the like is not ejected from the auxiliary water feeding channel opening 24 (see Fig. 2) and a state in which the degassed water or the like is ejected from the auxiliary water feeding channel opening 24 at a predetermined hydraulic pressure (see Fig. 3) are periodically repeated. Here, in the state in which the degassed water or the like is ejected from the auxiliary water feeding channel opening 24, living tissue in the vicinity of the ultrasound probe 17 is pressed by the hydraulic pressure. As a result, pressing force against the stomach wall 100 and the like by the ultrasound probe 17 is reduced without moving the ultrasound probe 17 relative to the stomach wall 100 and the like. That is, the pressing force against the stomach wall 100 and the like by the ultrasound probe 17 periodically switches between pressing force at the time when the degassed water or the like is not ejected from the auxiliary water feeding channel opening 24 (first pressing force) and pressing force at the time when the degassed water or the like is ejected from the auxiliary water feeding channel opening 24 (second pressing force weaker than the first pressing force). Then, by drive-controlling the ultrasound probe 17 (the ultrasound transducers 17a) each time the pressing force against the stomach wall 100 and the like by the ultrasound probe 17 switches between the first pressing force and the second pressing force, the ultrasound observation apparatus 50 acquires an ultrasound signal in each pressing state.

According to such an embodiment, by providing the ultrasound probe 17 arranged at the distal end rigid portion 10, the auxiliary water feeding channel opening 24 through which fluid is ejected from the vicinity of the ultrasound probe 17 in the projecting direction of the ultrasound probe 17, the auxiliary water feeding channel 47 communicating with the auxiliary water feeding channel opening 24, and the adjustment valve 48 for variably adjusting the flow rate of the fluid flowing through the auxiliary water feeding channel 47 among two or more states, it is possible to obtain a favorable elastography image by a simple configuration without upsizing the distal end portion.

That is, by adopting the configuration in which living tissue in the vicinity of the ultrasound probe 17 is pressed by the hydraulic pressure of the fluid ejected from the auxiliary water feeding channel opening 24, it is possible to obtain a favorable elastography image by the simple configuration without providing a pressing mechanism or the like for mechanically pressing a body cavity at the distal end rigid portion 10 of the endoscope 2.

In this case, by using the auxiliary water feeding channel opening 24 arranged at the position offset from the scanning area As of the ultrasound probe 17 as a fluid ejection nozzle, it is possible to reduce influence of the ejected fluid such as degassed water on an ultrasound signal.

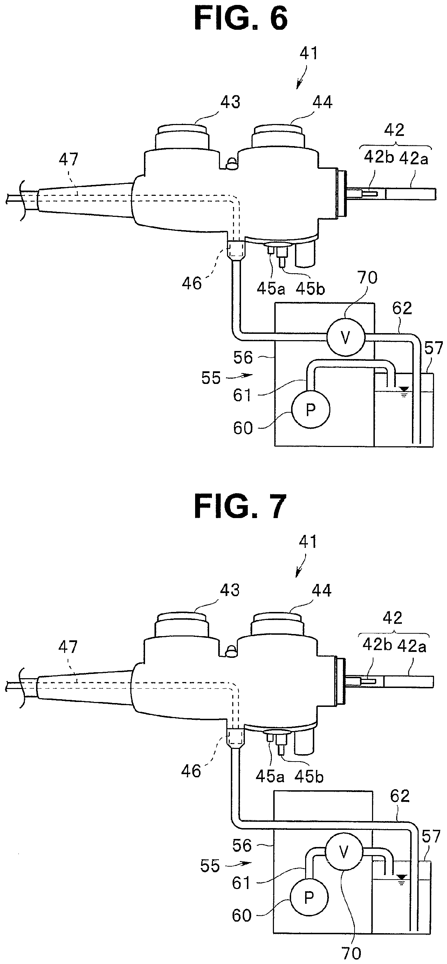

Next, Fig. 6 relates to a second embodiment of the present invention, and Fig. 6 is a schematic configuration diagram of a liquid feeding apparatus for ultrasound endoscope. Note that the present embodiment is different from the first embodiment described above mainly in a point that an adjustment valve 70 as a second flow rate adjusting portion is arranged on a liquid feeding apparatus for ultrasound endoscope 55 side, instead of the adjustment valve 48 as the first flow rate adjusting portion arranged on an endoscope 2 side. As for components and the like similar to those of the first embodiment described above, same reference numerals will be given, and description will be appropriately omitted.

As shown in Fig. 6, the adjustment valve 70 of the present embodiment is interposed, for example, in a middle of the auxiliary water feeding tube 62 in the pump unit 56.

The adjustment valve 70 is configured, for example, with a normally closed electromagnetic solenoid valve. The adjustment valve 70 can control the flow rate of the fluid flowing through the auxiliary water feeding channel 47 so that the flow rate is in an arbitrary state by the ultrasound observation apparatus 50 controlling a valve opening time period by an arbitrary duty ratio. More specifically, for example, by the valve opening time period being periodically duty-controlled by an arbitrary duty ratio, it is possible for the adjustment valve 70 to cause the flow rate of the fluid flowing through the auxiliary water feeding channel 47 to switch among a plurality of arbitrary states (for example, it is possible to cause the flow rate to periodically switch between arbitrary two states).

According to such an embodiment, it is possible to obtain operations and/or effects which are substantially similar to those of the first embodiment described above. In addition, in the present embodiment, by providing the adjustment valve 70 inside the liquid feeding apparatus for ultrasound endoscope 55, it is possible to obtain a favorable elastography image without making any change in design or the like in the endoscope 2 which is provided with the auxiliary water feeding channel opening 24 and the like.

Here, in the present embodiment, various modifications are possible as a configuration for performing flow rate adjustment by the liquid feeding apparatus for ultrasound endoscope 55.

For example, instead of the configuration in which the adjustment valve 70 is interposed in the auxiliary water feeding tube 62, it is also possible to interpose the adjustment valve 70 as the second flow rate adjusting portion in a middle of the pressurized tube 61 as shown in Fig. 7.

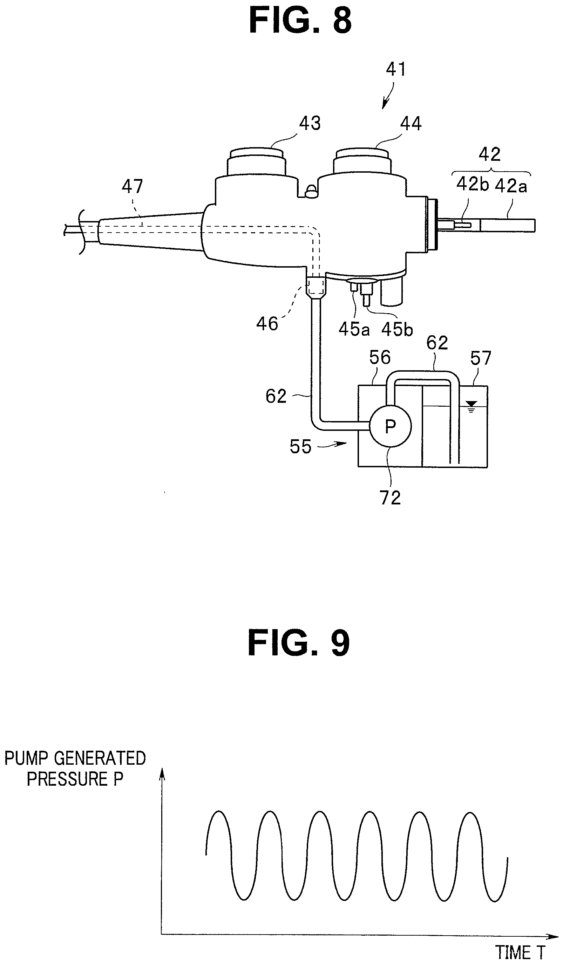

Further, for example, it is also possible to, instead of the pneumatic liquid feeding apparatus for ultrasound endoscope 55 which guides out liquid in the liquid feeding tank 57 utilizing air pressure generated by the pump unit 56, adopt a liquid feeding apparatus for ultrasound endoscope 55 of a type which directly pumps up the liquid in the liquid feeding tank 57 by a positive displacement pump 72 interposed in the middle of the auxiliary water feeding tube 62 in the pump unit 56, as shown in Fig. 8. In this case, for example, as shown in Fig. 9, since the positive displacement pump 72 constituting the pump unit 56 is originally such that generates predetermined pulsation at time of pumping up liquid, it is possible to periodically change the flow rate of the fluid flowing through the auxiliary water feeding channel 47 without using an adjustment valve. That is, in a modification shown in Fig. 8, the positive displacement pump 72 itself realizes functions as the fluid guiding-out portion and the second flow rate adjusting portion.

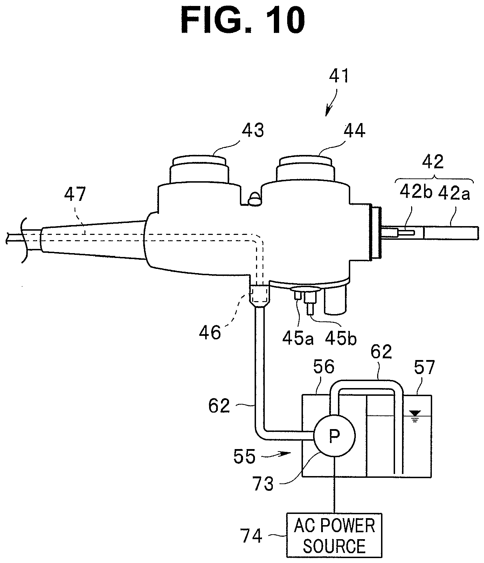

Otherwise, by adopting a DC pump 73 as a pump constituting the pump unit 56 instead of the positive displacement pump 72 and using an AC power source 74 as means for supplying power to the DC pump 73, for example, as shown in Fig. 10, it is possible to periodically change output power and periodically change the flow rate of the fluid flowing through the auxiliary water feeding channel 47 without using an adjustment valve. Furthermore, a power source control portion may be interposed between the DC pump 73 and the AC power source 74 to change a voltage period of the AC power source.

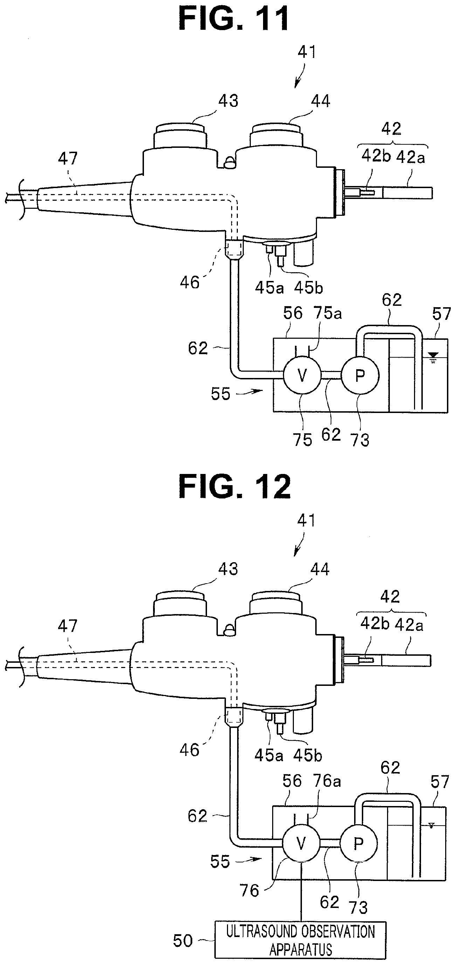

Further, it is also possible to interpose a relief valve 75 as the second flow rate adjusting portion in the middle of the auxiliary water feeding tube 62 on a downstream side of the DC pump 73, for example, as shown in Fig. 11 and cause a flow rate of degassed water or the like pressure-fed from the DC pump 73 to change by mechanical operation of the relief valve 75. That is, in such a configuration, the DC pump 73 is driven, for example, to pressure-feed fluid at a predetermined high pressure. Then, internal pressure of the auxiliary water feeding tube 62 between the DC pump 73 and a drain passage 75a increases and becomes a predetermined high pressure or higher, the relief valve 75 mechanically opens the drain passage 75a, and, thereby, hydraulic pressure in the auxiliary water feeding tube 62 between the DC pump 73 and the drain passage 75a decreases. On the other hand, when the internal pressure of the auxiliary water feeding tube 62 between the DC pump 73 and the drain passage 75a decreases and becomes lower than a predetermined low pressure by the drain passage 75a being opened, the relief valve 75 mechanically blocks the drain passage 75a, and, thereby, the internal pressure of the auxiliary water feeding tube 62 increases again. By the relief valve 75 repeating such operation, the flow rate of the fluid flowing through the auxiliary water feeding channel 47 is periodically changed.

Further, it is also possible to interpose a three-way valve 76 as the second flow rate adjusting portion in the middle of the auxiliary water feeding tube 62 on the downstream side of the DC pump 73, for example, as shown in Fig. 12 and cause the flow rate of the degassed water or the like pressure-fed from the DC pump 73 to change by controlling the three-way valve 76 by the ultrasound observation apparatus 50 or the like. That is, in such a configuration, the ultrasound observation apparatus 50 periodically connects the DC pump 73 to a downstream side of the auxiliary water feeding tube 62 and to a drain passage 76a alternately through control of the three-way valve 76. Thereby, the flow rate of the fluid flowing through the auxiliary water feeding channel 47 is periodically changed.

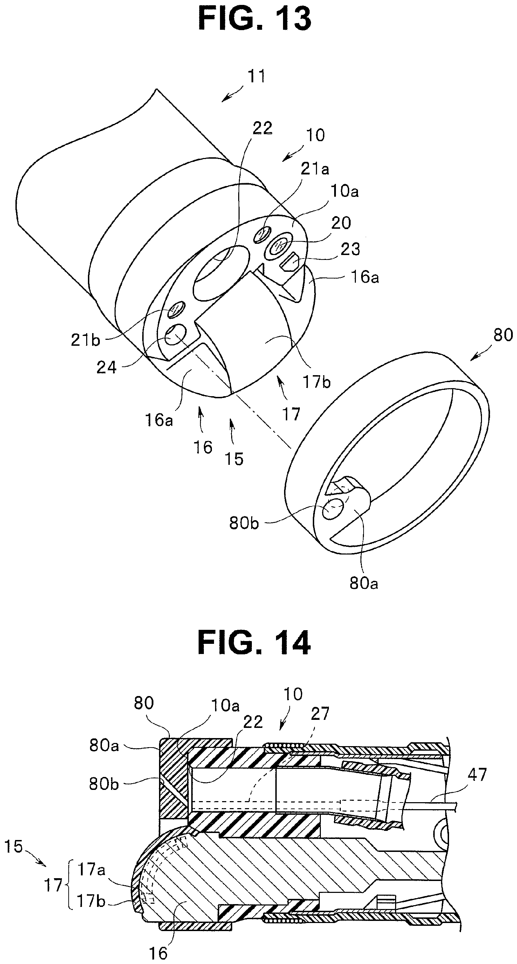

Next, Figs. 13 and 14 relate to a third embodiment of the present invention. Fig. 13 is an exploded perspective view showing a distal end portion and an adapter; and Fig. 14 is a cross-sectional view showing main portions of the distal end portion equipped with the adapter. Note that the present embodiment is different from the first and second embodiments described above mainly in a point that the distal end rigid portion 10 of the endoscope 2 is equipped with an adapter 80 to optimize an ejection direction of fluid. As for components and the like similar to those of the first and second embodiments described above, same reference numerals will be given, and description will be appropriately omitted.

As shown in Figs. 13 and 14, the distal end rigid portion 10 is equipped with a ring-shaped adapter 80 on a distal-end side outer circumference of the distal end rigid portion 10 by fitting or the like. At a position corresponding to the auxiliary water feeding channel opening 24 on an inner circumferential side of the adapter 80, a guiding projection 80a is provided, and a guide hole 80b communicating with the auxiliary water feeding channel opening 24 is made in the guiding projection 80a. For example, as shown in Fig. 14, the guide hole 80b functions as an opening portion for ejecting fluid and is inclined at a predetermined angle relative to an insertion axis direction of the distal end rigid portion 10. More specifically, the guide hole 80b has a component in the projecting direction of the ultrasound probe 17 and is inclined at the predetermined angle in a direction away from the ultrasound probe 17.

According to such a configuration, it is possible to adjust the ejection direction of the fluid such as degassed water supplied via the auxiliary water feeding channel opening 24 by fitting the adapter 80 having the guide hole 80b to an arbitrary direction. By setting the ejection direction of the fluid from the guide hole 80b to a direction having a component in the projecting direction of the ultrasound probe 17 and being away from the ultrasound probe 17, it is possible to cause a state of pressing living tissue to change while avoiding degassed water or the like from flowing in between the ultrasound probe 17 and the living tissue, and it becomes possible to acquire a more favorable ultrasound signal.

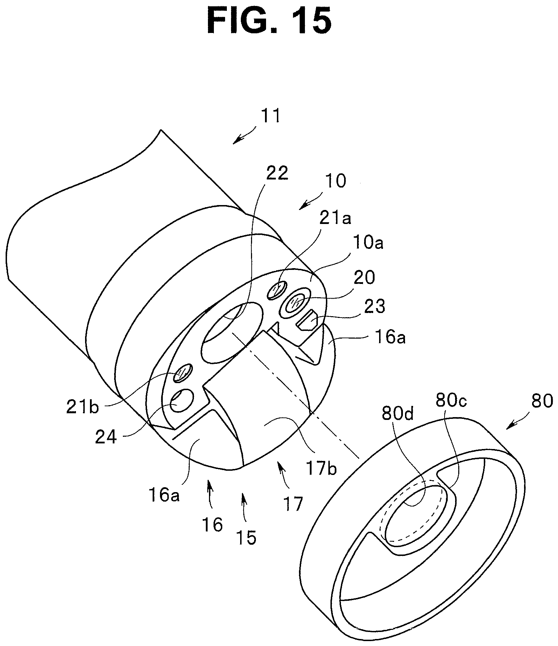

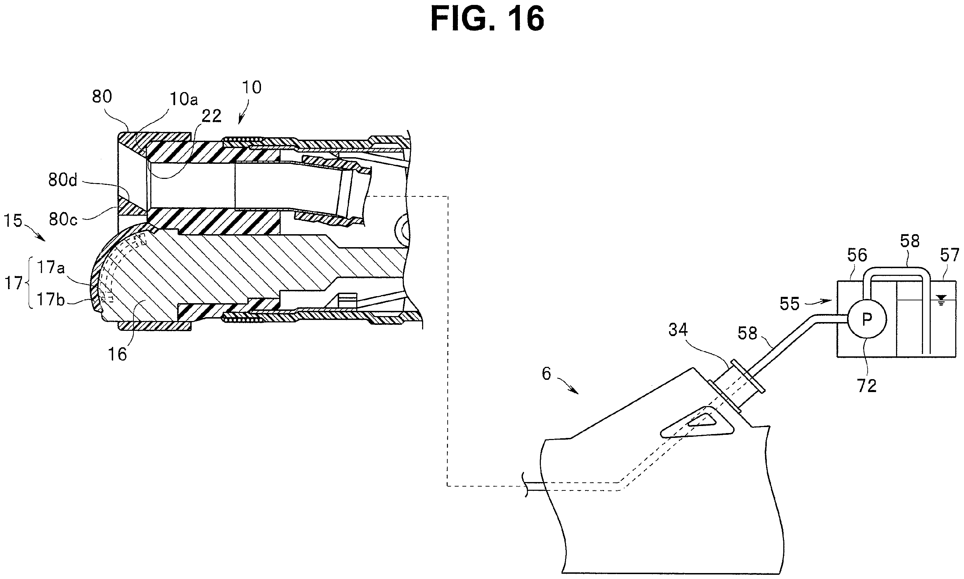

Here, by adopting the configuration in which the ejection direction of fluid is arbitrarily adjusted by the guide hole of the adapter 80 fitted to the distal end rigid portion 10, it is possible to use even the suction/forceps opening 22 or the like which is not offset from the scanning area as of the ultrasound probe 17 as an opening portion for ejection of fluid, for example, as shown in Figs. 15 and 16. In this case, the treatment instrument insertion channel communicating with the suction/forceps opening 22 functions as a fluid conduit. For example, as shown in Fig. 16, ejection of fluid via the suction/forceps opening 22 becomes possible by a water feeding tube 58 or the like of the liquid feeding apparatus for ultrasound endoscope 55 being inserted into the treatment instrument insertion port 34. Further, at a position corresponding to the suction/forceps opening 22 on the inner circumferential side of the adapter 80, a guiding projection 80c is provided, and a guide hole 80d communicating with the suction/forceps opening 22 is made in the guiding projection 80c. The guide hole 80d functions as an opening portion for ejecting fluid and is inclined at a predetermined angle relative to the insertion axis direction of the distal end rigid portion 10. More specifically, the guide hole 80d has a component in the projecting direction of the ultrasound probe 17 and is inclined at the predetermined angle in a direction away from the ultrasound probe 17. Note that, though detailed description for other matters will be omitted, it is possible to adopt a similar configuration, for example, for the air/water feeding nozzle 23.

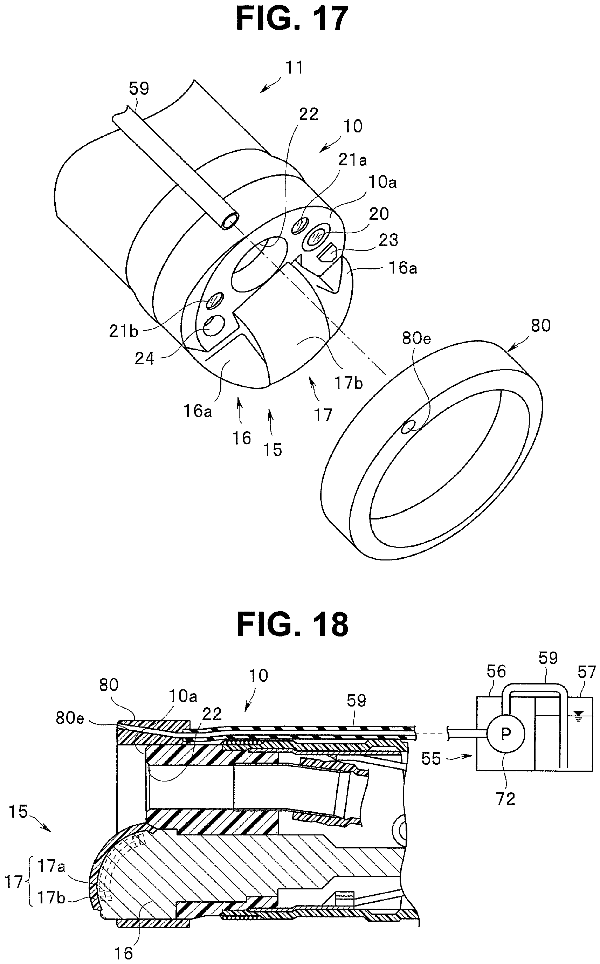

Furthermore, for example, as shown in Figs. 17 and 18, it is also possible to adopt a configuration in which an independent conduit 59 which is additionally provided for the insertion portion 5 of the endoscope 2 appropriately is used as a fluid conduit, and the independent conduit 59 is caused to communicate with a guide hole 80e of the adapter 80 fitted to the distal end rigid portion 10.

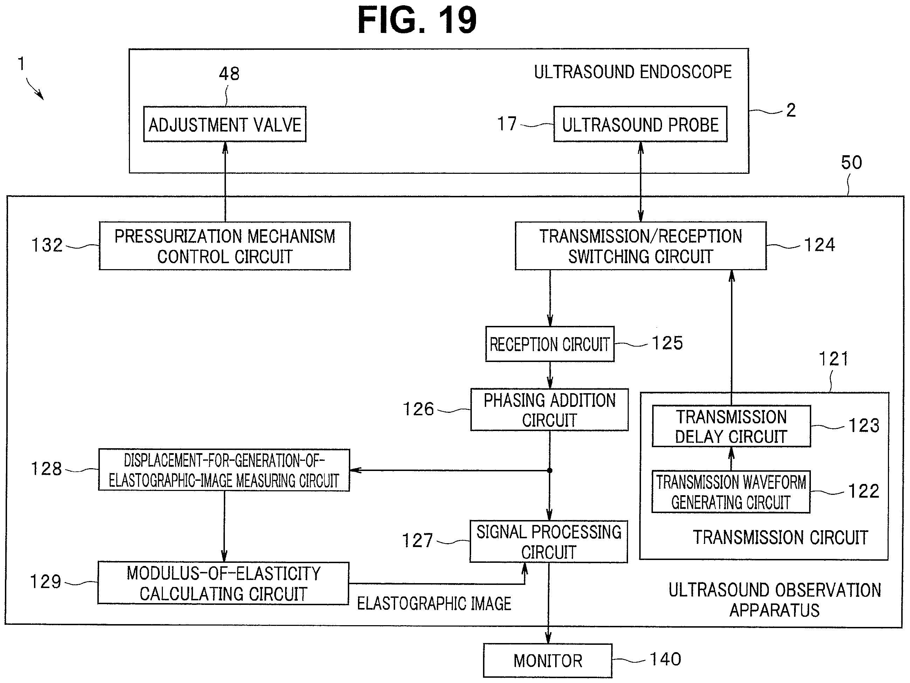

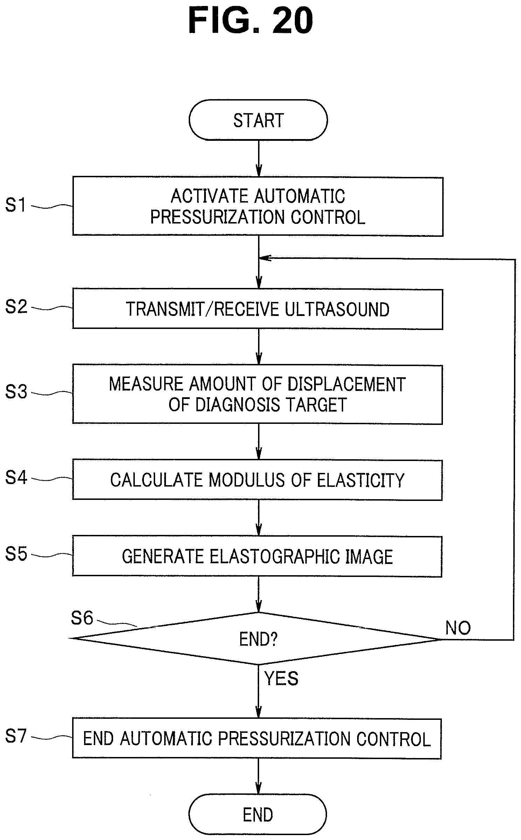

Next, Figs. 19 and 20 relate to a fourth embodiment of the present invention. Fig. 19 is a block diagram showing a configuration of an ultrasound endoscope system; and Fig. 20 is a flowchart showing an elastographic image generation process routine. Note that the present embodiment is such that describes a specific control example at a time of performing the elastography observation performed in the first embodiment described above, in detail. Therefore, as for components similar to those of the first embodiment described above, same reference numerals will be given, and description of the components will be appropriately omitted. Note that, though specific description will be omitted, similar control is, of course, applicable to the second and third embodiment described above.

The ultrasound observation apparatus 50 is provided with a transmission circuit 121, a transmission/reception switching circuit 124, a reception circuit 125, a phasing addition circuit 126, a signal processing circuit 127, a displacement-for-generation-of-elastographic-image measuring circuit 128, a modulus-of-elasticity calculating circuit 129 and a pressurization mechanism control circuit 132.

The transmission circuit 121 includes a transmission waveform generating circuit 122 and a transmission delay circuit 123.

The transmission waveform generating circuit 122 generates a signal waveform for driving each of the transducers 17a constituting the ultrasound probe 17 and outputs the signal waveform.

The transmission delay circuit 123 adjusts timing for driving each of the transducers 17a constituting the ultrasound probe 17. Thereby, a focus and direction of an ultrasound beam transmitted from the ultrasound probe 17 are controlled, and it is possible to cause ultrasound to converge to a desired position (depth).

The transmission/reception switching circuit 124 includes, for example, a multiplexer which sequentially selects a plurality of transducers for transmitting and receiving ultrasound; and the transmission/reception switching circuit 124 transmits a driving signal from the transmission circuit 121 to the ultrasound probe 17 and transmits an ultrasound signal (an echo signal) from the ultrasound probe 17 to the reception circuit 125.
The reception circuit 125 receives the ultrasound signal from the transmission/reception switching circuit 124 and performs, for example, processing such as amplification and conversion to a digital signal.

Thus, in the present embodiment, the transmission circuit 121 (the transmission waveform generating circuit 122 and the transmission delay circuit 123), the transmission/reception switching circuit 124 and the reception circuit 125 realize a function as a probe control portion.

The phasing addition circuit 126 delays an ultrasound signal to adjust a phase and then performs addition.

In an ultrasound diagnostic mode, the signal processing circuit 127 performs coordinate transformation or interpolation processing for an ultrasound signal from the phasing addition circuit 126 to create an ultrasound image as an image for display. Furthermore, in an elastographic image observation mode (an elastography image observation mode), the signal processing circuit 127 creates an image for display as for an elastographic image from the modulus-of-elasticity calculating circuit 129 or creates an image for display by superposing the elastographic image on an ultrasound image.

The displacement-for-generation-of-elastographic-image measuring circuit 128 is a displacement-for-elastographic-image measuring portion which measures an amount of displacement for an image of a subject (an amount of displacement for generating an elastographic image of the subject) based on an ultrasound signal.

The modulus-of-elasticity calculating circuit 129 is a modulus-of-elasticity calculating portion which calculates a modulus of elasticity of the subject based on the amount of displacement for image measured by the displacement-for-generation-of-elastographic-image measuring circuit 128. Since the modulus-of-elasticity calculating circuit 129 calculates a modulus of elasticity for each coordinate of the subject, a calculation result is an elastographic image on which moduli of elasticity are distributed on two-dimensional coordinates.

Thus, in the present embodiment, the phasing addition circuit 126, the signal processing circuit 127, the displacement-for-generation-of-elastographic-image measuring circuit 128 and the modulus-of-elasticity calculating circuit 129 realize a function as an elastographic image generating portion.

For example, when an instruction to the effect that elastography observation is to be started is made through an operation of the buttons/switches 33 by the user or the like (that is, when the elastographic image observation mode is selected), the pressurization mechanism control circuit 132 performs an automatic pressurization (pressure reduction) control process for the subject. That is, by performing valve opening control of the adjustment valve 48 at a predetermined duty ratio for each predetermined period and controlling an amount of ejection of fluid ejected from the auxiliary water feeding channel opening 24, through the valve opening control, the pressurization mechanism control circuit 132 indirectly causes a pressing state of the ultrasound probe 17 pressing the subject to change. It is desirable to perform such control performed for the adjustment valve 48 in synchronization of spontaneous displacement such as beats of the subject. For example, in the present embodiment in which pressing force against the subject by the ultrasound probe 17 is reduced by ejection of fluid, it is desirable that the control valve 48 is controlled so that the amount of ejection of the fluid is smallest at timing when the spontaneous displacement of the subject is the largest (otherwise, so that the amount of ejection of the fluid becomes zero).

Thus, in the present embodiment, the pressurization mechanism control circuit 132 realizes a function as a pressing control portion.

A monitor 140 displays an image for display from the signal processing circuit 127.

Next, Fig. 20 is a flowchart showing an elastographic image generation process routine.

When the ultrasound endoscope system 1 is set to the elastographic image observation mode, the process shown in Fig. 20 is started.

Then, first, an automatic pressurization control process in the pressurization mechanism control circuit 132 is activated (step S1).

Then, transmission/reception of ultrasound to and from the ultrasound probe 17 is performed (step S2), and an amount of displacement (an amount of displacement for image) of a subject to be a diagnosis target is measured by the displacement-for-generation-of-elastographic-image measuring circuit 128 (step S3).

Next, the modulus-of-elasticity calculating circuit 129 calculates a modulus of elasticity of the subject for each coordinate of the subject, based on the amount of displacement for image measured at step S3 (step S4).

The calculated moduli of elasticity is transmitted to the signal processing circuit 127 together with coordinates and configured as an elastographic image for display (step S5). The elastographic image is further superposed on an ultrasound image as necessary to create an image for display, and the image for display is displayed on the monitor 140.

After that, it is judged whether the process is to be ended or not (step S6). If the process is not to be ended, the process returns to step S2 to generate an elastographic image for a next frame, and the process as described above is repeated.

On the other hand, if it is judged that the process is to be ended, the automatic pressurization control process by the pressurization mechanism control circuit 132 is caused to end (step S7), and then the elastographic image generation process is ended.

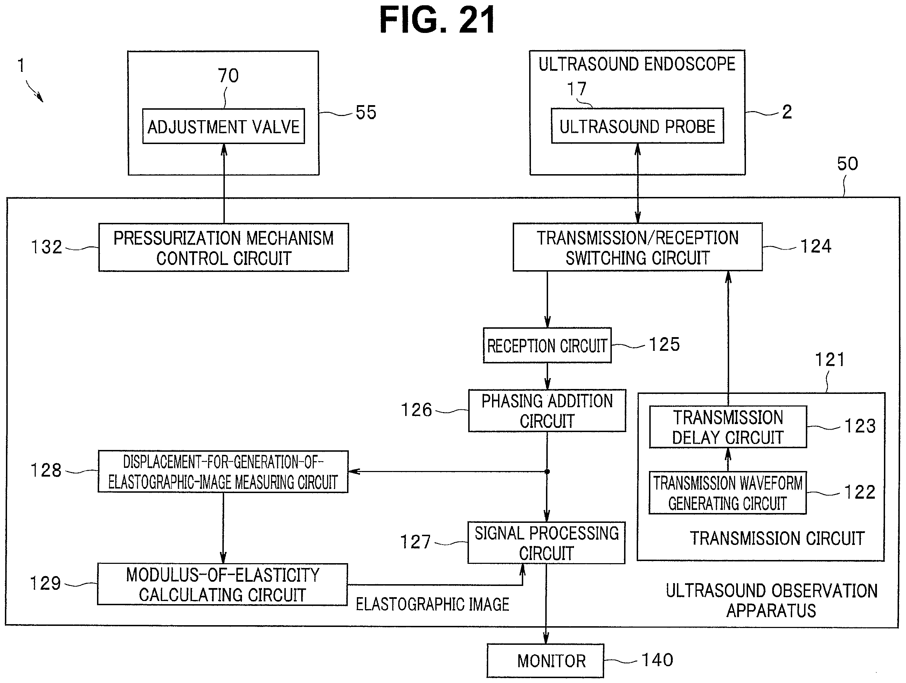

Here, though detailed description is omitted, the present embodiment can be, of course, applied to the configuration shown in the second embodiment, for example, as shown in Fig. 21. Note that the present invention is not limited to each of the embodiments described above, and various modifications and changes are possible. The modifications and changes are also within a technical scope of the present invention. For example, though description has been made on an example of the case of performing elastography observation by the ultrasound endoscope system 1 to perform examination of a tumor/lymph node metastasis of the pancreas 101 and the like in the embodiments described above, the elastography observation using the present invention can be applied to various examinations for chronic pancreatitis, a tumor/lymph node metastasis of a liver, hepatic cirrhosis, a tumor/lymph node metastasis of a mediastinum (an esophagus), a tumor/lymph node metastasis of a prostate, and the like.

Further it is, of course, possible to appropriately combine components of the respective embodiments and respective modifications described above.

This application is filed, claiming priority based on Japanese Patent Application No. 2014-169442 filed to Japan on August 22, 2014.

## Claims

1. An ultrasound endoscope system (1) comprising:
an ultrasound endoscope (2) for elastography comprising:
a long and flexible insertion portion (5) configured to be inserted into a subject;
a distal end portion (10) arranged at a distal end of the insertion portion (5);
an ultrasound observation portion (17) arranged at the distal end portion (10);
an opening portion (22, 23, 24, 80b, 80d, 80e) enabling fluid to be ejected from a vicinity of the ultrasound observation portion (17) in a projecting direction of the ultrasound observation portion (17) for pressing living tissue;
a fluid conduit (47, 59) communicating with the opening portion (22, 23, 24, 80b, 80d, 80e); and
a flow rate adjusting portion (48) configured to cause the ejection of the fluid to periodically change by switching a flow rate of the fluid flowing through the fluid conduit (47, 59) between two states at an arbitrary duty ratio,
**characterized in that** the ultrasound endoscope system (1) further
comprises
a pressing control portion (132) configured to control the flow rate through the flow rate adjusting portion (48) so that the flow rate changes at a predetermined duty ratio;
a probe control portion (121, 122, 123, 124,125) configured to drive and control the ultrasound observation portion (17) in two pressing states controlled by the pressing control portion (132) to acquire an ultrasound signal of the living tissue; and
an elastographic image generating portion (126,127,128,129) configured to measure an amount of displacement of the subject based on the ultrasound signal and to generate an elastographic image showing distribution of moduli of elasticity for respective coordinates of the subject based on the amount of displacement.

2. The ultrasound endoscope system (1) according to claim 1, wherein the ultrasound endoscope (2) comprises an observation window arranged at the distal end portion (10) and a suction and forceps opening (22) for guiding out an air/water feeding nozzle for ejecting the fluid toward the observation window and a treatment instrument such as a puncture needle, and
the opening portion is an auxiliary water feeding channel opening (24) provided separately from the suction and forceps opening.

3. The ultrasound endoscope system (1) according to claim 1, wherein the opening portion of the ultrasound endoscope (2) is provided at a position offset relative to a scanning area of the ultrasound observation portion (17).

4. The ultrasound endoscope system (1) according to claim 1, wherein the opening portion of the ultrasound endoscope (2) is formed in an adapter (80) fitted to the distal end portion (10).

5. An ultrasound endoscope system (1) comprising:
an ultrasound endoscope for elastography (2) comprising:
a long and flexible insertion portion (5) configured to be inserted into a subject;
a distal end portion (10) arranged at a distal end of the insertion portion (5);
an ultrasound observation portion (17) arranged at the distal end portion (10);
an opening portion (22, 23, 24, 80b, 80d, 80e) enabling fluid to be ejected from a vicinity of the ultrasound observation portion (17) in a projecting direction of the ultrasound observation portion (17) for pressing living tissue; and
a fluid conduit (47, 59) communicating with the opening portion (22, 23, 24, 80b, 80d, 80e);
a liquid feeding apparatus for ultrasound endoscope comprising:
a fluid guiding-out portion (60, 72) configured to guide the fluid into the fluid conduit; and
a flow rate adjusting portion (70) configured to cause
the ejection of the fluid to periodically change by switching a flow rate of the fluid guided out from the fluid guiding-out portion (60, 72) between two states at an arbitrary duty ratio,
**characterized by**
a pressing control portion (132) configured to control the flow rate through the flow rate adjusting portion (70) so that the flow rate changes at a predetermined duty ratio;
a probe control portion (121, 122, 123,124,125) configured to drive and control the ultrasound observation portion in two pressing states controlled by the pressing control portion (132) to acquire an ultrasound signal of the living tissue; and
an elastographic image generating portion (126,127, 128,129) configured to measure an amount of displacement of the subject based on the ultrasound signal and to generate an elastographic image showing distribution of moduli of elasticity for respective coordinates of the subject based on the amount of displacement.

## Patentansprüche

1. Ultraschallendoskopsystem (1), umfassend:
ein Ultraschallendoskop (2) für Elastographie, umfassend:
einen langen und flexiblen Einführabschnitt (5), der zur Einführung in ein Subjekt ausgelegt ist;
einen distalen Endabschnitt (10), der an einem distalen Ende des Einführabschnitts (5) angeordnet ist;
einen Ultraschall-Beobachtungsabschnitt (17), der am distalen Endabschnitt (10) angeordnet ist;
einen Öffnungsabschnitt (22, 23, 24, 80b, 80d, 80e), der das Ausstoßen von Flüssigkeit aus einer Nähe des Ultraschall-Beobachtungsabschnitts (17) in einer Projektionsrichtung des Ultraschall-Beobachtungsabschnitts (17) zum Drücken auf lebendes Gewebe ermöglicht;
eine Flüssigkeitsleitung (47, 59), die mit dem Öffnungsabschnitt (22, 23, 24, 80b, 80d, 80e) in Verbindung steht; und
einen Flussraten-Einstellabschnitt (48), der dazu ausgelegt ist, eine regelmäßige Änderung des Ausstoßens der Flüssigkeit zu bewirken, indem eine Flussrate der Flüssigkeit, die durch die Flüssigkeitsleitung (47, 59) strömt, zwischen zwei Zuständen mit einer beliebigen relativen Einschaltdauer umgeschaltet wird,
**dadurch gekennzeichnet, dass** das Ultraschallendoskopsystem (1) weiterhin einen Drück-Kontrollabschnitt (132) umfasst, der zur Steuerung der Flussrate durch den Flussraten-Einstellabschnitt (48) ausgelegt ist, so dass sich die Flussrate mit einer vorgegebenen relativen Einschaltdauer ändert;
einen Sondenkontrollabschnitt (121, 122, 123, 124, 125), der zum Antrieb und zur Steuerung des Ultraschall-Beobachtungsabschnitts (17) mit zwei Drück-Zuständen ausgelegt ist, die durch den Drück-Kontrollabschnitt (132) gesteuert werden, um ein Ultraschallsignal des lebenden Gewebes zu erfassen; und
einen Elastographiebild-Erzeugungsabschnitt (126, 127, 128, 129), der zur Messung eines Ausmaßes der Verlagerung des Subjekts auf der Grundlage des Ultraschallsignals sowie zur Erzeugung eines Elastographiebildes ausgelegt ist, das die Verteilung von Elastizitätsmoduln für entsprechende Koordinaten des Subjekts auf der Grundlage des Ausmaßes der Verlagerung zeigt.

2. Ultraschallendoskopsystem (1) nach Anspruch 1, wobei das Ultraschallendoskop (2) ein Beobachtungsfenster umfasst, das am distalen Endabschnitt (10) angeordnet ist, sowie eine Absaug- und Zangenöffnung (22) zum Herausführen einer Luft-/Wasserzuführungsdüse zum Ausstoßen der Flüssigkeit zum Beobachtungsfenster und eines Behandlungsinstruments, wie eine Punktionsnadel, und
der Öffnungsabschnitt eine Hilfs-Wasserzufuhrkanalöffnung (24) ist, die separat von der Absaug- und Zangenöffnung bereitgestellt wird.

3. Ultraschallendoskopsystem (1) nach Anspruch 1, wobei der Öffnungsabschnitt des Ultraschallendoskops (2) an einer Position bereitgestellt wird, die relativ zu einem Scanbereich des Ultraschall-Beobachtungsabschnitts (17) versetzt ist.

4. Ultraschallendoskopsystem (1) nach Anspruch 1, wobei der Öffnungsabschnitt des Ultraschallendoskops (2) in einem Adapter (80) ausgebildet ist, der am distalen Endabschnitt (10) angebracht ist.

5. Ultraschallendoskopsystem (1), umfassend:
ein Ultraschallendoskop für Elastographie (2), umfassend:
einen langen und flexiblen Einführabschnitt (5), der zur Einführung in ein Subjekt ausgelegt ist;
einen distalen Endabschnitt (10), der an einem distalen Ende des Einführabschnitts (5) angeordnet ist;
einen Ultraschall-Beobachtungsabschnitt (17), der am distalen Endabschnitt (10) angeordnet ist;
einen Öffnungsabschnitt (22, 23, 24, 80b, 80d, 80e), der das Ausstoßen von Flüssigkeit aus einer Nähe des Ultraschall-Beobachtungsabschnitts (17) in einer Projektionsrichtung des Ultraschall-Beobachtungsabschnitts (17) zum Drücken auf lebendes Gewebe ermöglicht; und
eine Flüssigkeitsleitung (47, 59), die mit dem Öffnungsabschnitt (22, 23, 24, 80b, 80d, 80e) in Verbindung steht;
eine Flüssigkeitszufuhr-Vorrichtung für ein Ultraschallendoskop, umfassend:
einen Flüssigkeits-Herausführungsabschnitt (60, 72), der zum Hineinführen der Flüssigkeit in die Flüssigkeitsleitung ausgelegt ist; und
einen Flussraten-Einstellabschnitt (70), der dazu ausgelegt ist, eine regelmäßige Änderung des Ausstoßens der Flüssigkeit zu bewirken, indem eine Flussrate der Flüssigkeit, die aus dem Flüssigkeits-Herausführungsabschnitt (60, 72) herausgeführt wird, zwischen zwei Zuständen mit einer beliebigen relativen Einschaltdauer umgeschaltet wird, **gekennzeichnet durch**
einen Drück-Kontrollabschnitt (132), der zur Steuerung der Flussrate durch den Flussraten-Einstellabschnitt (70) ausgelegt ist, so dass sich die Flussrate mit einer vorgegebenen relativen Einschaltdauer ändert;
einen Sondenkontrollabschnitt (121, 122, 123, 124, 125), der zum Antrieb und zur Steuerung des Ultraschall-Beobachtungsabschnitts mit zwei Drück-Zuständen ausgelegt ist, die durch den Drück-Kontrollabschnitt (132) gesteuert werden, um ein Ultraschallsignal des lebenden Gewebes zu erfassen; und
einen Elastographiebild-Erzeugungsabschnitt (126, 127, 128, 129), der zur Messung eines Ausmaßes der Verlagerung des Subjekts auf der Grundlage des Ultraschallsignals sowie zur Erzeugung eines Elastographiebildes ausgelegt ist, das die Verteilung von Elastizitätsmoduln für entsprechende Koordinaten des Subjekts auf der Grundlage des Ausmaßes der Verlagerung zeigt.

## Revendications

1. Système d'endoscope à ultrasons (1) comprenant :
un endoscope à ultrasons (2) pour élastographie comprenant :
une partie d'insertion longue et flexible (5) configurée pour être insérée dans un sujet ;
une partie d'extrémité distale (10) agencée à une extrémité distale de la partie d'insertion (5) ;
une partie d'observation à ultrasons (17) agencée à la partie d'extrémité distale (10) ;
une partie d'ouverture (22, 23, 24, 80b, 80d, 80e) permettant à du fluide d'être éjecté à partir d'un voisinage de la partie d'observation à ultrasons (17) dans une direction de projection de la partie d'observation à ultrasons (17) pour presser du tissu vivant ;
un conduit de fluide (47, 59) communiquant avec la partie d'ouverture (22, 23, 24, 80b, 80d, 80e) ; et
une partie d'ajustement de débit (48) configurée pour amener l'éjection du fluide à changer périodiquement par commutation d'un débit du fluide s'écoulant à travers le conduit de fluide (47, 59) entre deux états à un rapport cyclique arbitraire, **caractérisé par le fait que** le système d'endoscope à ultrasons (1) comprend en outre :
une partie de commande de pression (132) configurée pour commander le débit par l'intermédiaire de la partie d'ajustement de débit (48) de telle sorte que le débit change à un rapport cyclique prédéterminé ;
une partie de commande de sonde (121, 122, 123, 124, 125) configurée pour piloter et commander la partie d'observation à ultrasons (17) dans deux états de pression commandés par la partie de commande de pression (132) pour acquérir un signal ultrasonore du tissu vivant ; et
une partie de génération d'image élastographique (126, 127, 128, 129) configurée pour mesurer une quantité de déplacement du sujet sur la base du signal ultrasonore et générer une image élastographique montrant une distribution de modules d'élasticité pour des coordonnées respectives du sujet sur la base de la quantité de déplacement.

2. Système d'endoscope à ultrasons (1) selon la revendication 1, dans lequel l'endoscope à ultrasons (2) comprend une fenêtre d'observation agencée à la partie d'extrémité distale (10) et une ouverture d'aspiration et de pince (22) pour guider en sortie une buse d'alimentation en air/eau pour éjecter le fluide vers la fenêtre d'observation et un instrument de traitement tel qu'une aiguille de ponction, et
la partie d'ouverture est une ouverture de canal d'alimentation en eau auxiliaire (24) prévue séparément de l'ouverture d'aspiration et de pince.

3. Système d'endoscope à ultrasons (1) selon la revendication 1, dans lequel la partie d'ouverture de l'endoscope à ultrasons (2) est prévue à une position décalée par rapport à une zone de balayage de la partie d'observation à ultrasons (17).

4. Système d'endoscope à ultrasons (1) selon la revendication 1, dans lequel la partie d'ouverture de l'endoscope à ultrasons (2) est formée dans un adaptateur (80) monté sur la partie d'extrémité distale (10).

5. Système d'endoscope à ultrasons (1) comprenant :
un endoscope à ultrasons pour élastographie (2) comprenant :
une partie d'insertion longue et flexible (5) configurée pour être insérée dans un sujet ;
une partie d'extrémité distale (10) agencée à une extrémité distale de la partie d'insertion (5) ;
une partie d'observation à ultrasons (17) agencée à la partie d'extrémité distale (10) ;
une partie d'ouverture (22, 23, 24, 80b, 80d, 80e) permettant à du fluide d'être éjecté à partir d'un voisinage de la partie d'observation à ultrasons (17) dans une direction de projection de la partie d'observation à ultrasons (17) pour presser du tissu vivant ; et
un conduit de fluide (47, 59) communiquant avec la partie d'ouverture (22, 23, 24, 80b, 80d, 80e) ;
un appareil d'alimentation en liquide pour endoscope à ultrasons comprenant :
une partie de guidage de sortie de fluide (60, 72) configurée pour guider le fluide dans le conduit de fluide ; et
une partie d'ajustement de débit (70) configurée pour amener l'éjection du fluide à changer périodiquement par commutation d'un débit du fluide guidé en sortie à partir de la partie de guidage de sortie de fluide (60, 72) entre deux états à un rapport cyclique arbitraire,
**caractérisé par**
une partie de commande de pression (132) configurée pour commander le débit par l'intermédiaire de la partie d'ajustement de débit (70) de telle sorte que le débit change à un rapport cyclique prédéterminé ;
une partie de commande de sonde (121, 122, 123, 124, 125) configurée pour piloter et commander la partie d'observation à ultrasons dans deux états de pression commandés par la partie de commande de pression (132) pour acquérir un signal ultrasonore du tissu vivant ; et
une partie de génération d'image élastographique (126, 127, 128, 129) configurée pour mesurer une quantité de déplacement du sujet sur la base du signal ultrasonore et générer une image élastographique montrant une distribution de modules d'élasticité pour des coordonnées respectives du sujet sur la base de la quantité de déplacement.
